# EUROPEAN PATENT SPECIFICATION

(11) **EP 3 395 248 B1**
(45) Date of publication and mention of the grant of the patent: **17.02.2021**
(21) Application number: 18169436.5
(22) Date of filing: 26.04.2018
(51) Int. Cl.: A61B 5/06, G01B 7/004, G01L 1/14

(54) **MECHANICAL FORCE SENSOR BASED ON EDDY CURRENT SENSING**
MECHANISCHER KRAFTSENSOR BASIEREND AUF WIRBELSTROMERFASSUNG
CAPTEUR DE FORCE MÉCANIQUE SUR LA BASE DE DÉTECTION DE COURANT DE FOUCAULT

(30) Priority: 27.04.2017 US 201762490786 P; 29.03.2018 US 201815940563
(43) Date of publication of application: 31.10.2018
(73) Proprietor: Biosense Webster (Israel) Ltd., Yokneam 2066717 (IL)
(72) Inventor: SHAMELI, Ehsan, Irvine, CA California 92618 (US); EBRAHIMI, Babak, Irvine, CA California 92618 (US)
(74) Representative: Small, Gary James

(56) References cited:
- EP-A1- 2 888 997
- EP-A2- 2 000 789

## Description

### FIELD OF THE INVENTION

The present invention relates generally to the field of medical devices, and specifically to mechanical force sensors for catheters.

### BACKGROUND

Eddy currents, also known as Foucault currents, are closed loops of electrical current induced in a conductor upon exposure of the conductor to a varying magnetic field, or upon oscillation of the conductor in a static magnetic field.

US Patent 7,984,659, describes a measurement device that can detect a degree of bending of a linear body with a sensor when compressive force in a direction of longitudinal axis is applied to the linear body as a result of contact of a tip end of the linear body with an obstacle. Then, the detected degree of bending of the linear body is converted to compressive force in the direction of longitudinal axis applied to the linear body based on predetermined correlation between the degree of bending and the compressive force, so that presence of an obstacle in a direction of travel of the linear body can be sensed based on increase in the compressive force.

EP 2 888 997 A1 discloses adaptive fluoroscope location for the application of field compensation. Compensation for a field-perturbing element in a magnetic tracking system (10) for locating a probe (22) is achieved by creating a reaction field model while the field-perturbing element is in known positions. A magnetic field location sensor (26) is disposed between the field perturbing element and magnetic field generators of the tracking system. Magnetic field readings from the location sensor and from a magnetic field sensor (46, 40, 42, 44) on the probe are taken while the field-perturbing element is present. The position of the field-perturbing element is estimated from the location sensor readings, and a predicted reaction field calculated from the reaction field model. A compensated measurement is obtained by subtracting the predicted reaction field from the field detected by the location sensor. The readings from the probe sensor are adjusted using the compensated measurement in order to calculate the true position of the probe.

### SUMMARY OF THE INVENTION

There is provided, in accordance with the present invention, an apparatus that includes a catheter configured for insertion into a body of a subject, the catheter including a flexible distal portion configured to flex in response to a mechanical force applied to the catheter. The apparatus further includes a conducting element, held by the flexible distal portion of the catheter such that a position of the conducting element changes as the flexible distal portion flexes. The apparatus further includes at least one transmitting coil, disposed within the catheter proximally to the conducting element, configured to generate an alternating magnetic field that induces, in the conducting element, eddy currents that vary with the position of the conducting element. The apparatus further includes one or more receiving coils, disposed within the catheter proximally to the conducting element, configured to output respective signals responsively to a superposition of (i) the magnetic field generated by the transmitting coil, and (ii) a secondary magnetic field generated by the eddy currents.

In some embodiments, the conducting element is held within the flexible distal portion of the catheter.

In some embodiments, the conducting element is affixed to a distal end of the flexible distal portion of the catheter.

In some embodiments, the catheter further includes a tube, and the flexible distal portion of the catheter includes a flexible distal portion of the tube that is of enhanced flexibility relative to a more proximal portion of the tube.

In some embodiments, the catheter further includes a tube, and the flexible distal portion of the catheter includes a cylindrical element that extends distally from the tube and is of enhanced flexibility relative to the tube.

In some embodiments, the cylindrical element extends distally from the tube for a distance of between 0.5 and 2 mm.

In some embodiments, the flexible distal portion of the catheter is of enhanced flexibility by virtue of being shaped to define at least one groove.

In some embodiments, the at least one groove includes a helical groove.

In some embodiments, the apparatus further includes a processor, configured to ascertain a magnitude and a direction of the mechanical force in response to the respective signals output by the receiving coils.

In some embodiments, the apparatus further includes an electronic interface, the processor is further configured to generate a digital signal, and the electronic interface is configured to convert the digital signal to an analog signal which, when applied across the transmitting coil, causes the transmitting coil to generate the alternating magnetic field.

In some embodiments, the receiving coils are disposed at least partly within the transmitting coil.

In some embodiments, the transmitting coil is wrapped around the receiving coils.

In some embodiments, the conducting element includes a plate.

In some embodiments, the conducting element includes a tube.

In some embodiments, the conducting element is shaped to define a central aperture.

In some embodiments, the apparatus further includes:
an ablation electrode, coupled distally to the flexible distal portion of the catheter, configured to pass ablating currents into tissue of the subject while the catheter is inside the body of the subject; and
a fluid-delivery tube that passes through the central aperture and is configured to deliver fluid to the ablation electrode.

In some embodiments, the apparatus further includes:
at least one physiological sensor, coupled to the catheter distally to the flexible distal portion of the catheter; and
at least one wire that passes through the central aperture and is connected to the physiological sensor.

There is further provided, in accordance with some embodiments of the present invention, a method that includes, using at least one transmitting coil disposed within a catheter inside a body of a subject, generating an alternating magnetic field that induces eddy currents in a conducting element that is held, distally to the transmitting coil, by a flexible distal portion of the catheter such that a position of the conducting element changes as the flexible distal portion flexes in response to a mechanical force applied to the catheter, the eddy currents varying with the position of the conducting element. The method further includes, using one or more receiving coils disposed within the catheter proximally to the conducting element, outputting respective signals responsively to a superposition of (i) the magnetic field generated by the transmitting coil, and (ii) a secondary magnetic field generated by the eddy currents. The method further includes, using a processor, ascertaining a magnitude and a direction of the mechanical force, in response to the respective signals output by the receiving coils.

The present invention will be more fully understood from the following detailed description of embodiments thereof, taken together with the drawings, in which:

### BRIEF DESCRIPTION OF THE DRAWINGS

Fig. 1 is a schematic illustration of apparatus comprising a catheter configured for insertion into the body of a subject, in accordance with some embodiments of the present invention; and
Figs. 2-3 show various components contained within the catheter of Fig. 1, in accordance with some embodiments of the present invention.

### DETAILED DESCRIPTION OF EMBODIMENTS

### OVERVIEW

In some applications, a catheter is inserted into the body of a subject, and is subsequently used to acquire information from the body, and/or to treat the body. For example, a catheter may be inserted into the heart of a subject, and subsequently used to ablate tissue of the heart.

In such applications, it may be helpful to measure the mechanical force that is applied to the distal end of the catheter. For example, based on the magnitude of this force, the presence or absence of contact of the distal end with tissue of the subject may be ascertained. Moreover, since the force applied to the catheter by the tissue is equivalent to the force applied to the tissue by the catheter, the contact pressure applied to the tissue may be identified. Furthermore, based on the direction of the measured force, the orientation of the distal end may be ascertained.

Embodiments of the present invention therefore provide a catheter that comprises a mechanical force sensor at its distal end. The sensor comprises a conducting plate (or any other suitable conducting element), and further comprises a transmitting coil and a plurality of receiving coils, which are disposed proximally to the conducting plate. An alternating current is passed through the transmitting coil, causing an alternating magnetic field to be generated. This magnetic field induces eddy currents in the conducting plate, which generate a secondary magnetic field that is detected by the receiving coils. (For simplicity, it may be said that the receiving coils detect the eddy currents.) As mechanical forces are applied (e.g., by the tissue) to the distal end of the catheter, the position and/or orientation of the conducting plate relative to the transmitting coil may change, such that the receiving coils sense variations in the eddy currents. By analyzing these variations, a processor may ascertain the magnitude and direction of the mechanical forces.

Typically, the distal portion of the catheter that holds the conducting plate is of enhanced (i.e., increased) flexibility, relative to other portions of the catheter. For example, the distal portion may be shaped to define a helical groove that imparts enhanced flexibility. This enhanced flexibility amplifies the changes to the position and/or orientation of the conducting plate caused by the mechanical forces acting on the distal end of the catheter.

Advantageously, the positioning of the transmitting and receiving coils proximally to the flexible distal portion of the catheter, at approximately the same axial position within the catheter, may simplify the manufacturing process for the apparatus. For example, the transmitting coil may be wrapped around the receiving coils to form an integrated coil package, and then the coil package may be installed, straightforwardly, within the catheter. In contrast, if the transmitting coil were positioned distally to the flexible distal portion of the catheter, the manufacturing process would require separate installations of the receiving coils and transmitting coil, the latter installation being complicated by the need to run wires, which connect the transmitting coil to the proximal end of the catheter, through the flexible distal portion of the catheter. Furthermore, since the aforementioned wires are typically relatively thin, flexion of the distal portion of the catheter might cause the wires to tear during usage of the catheter.

### APPARATUS DESCRIPTION

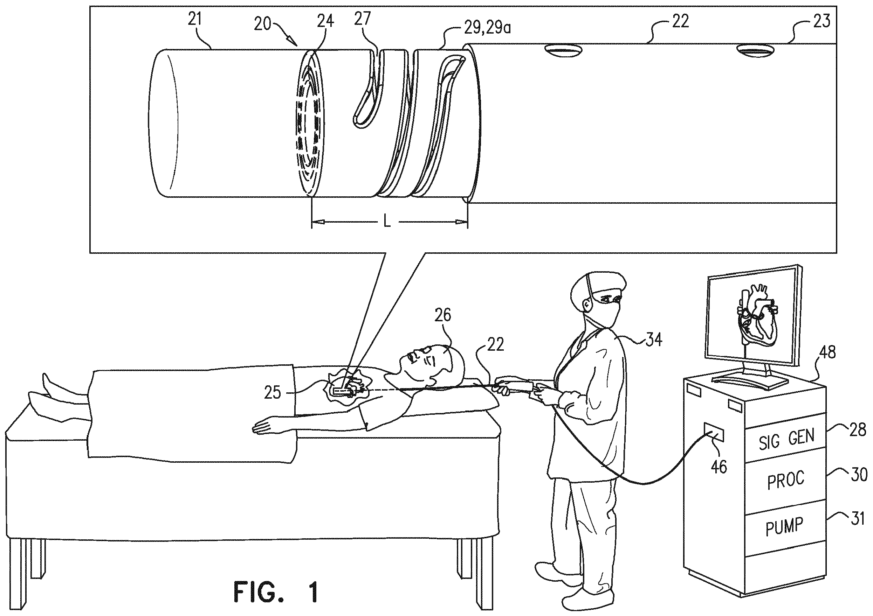

Reference is initially made to Fig. 1, which is a schematic illustration of apparatus 20 comprising a catheter 22 configured for insertion into the body of a subject, in accordance with some embodiments of the present invention.

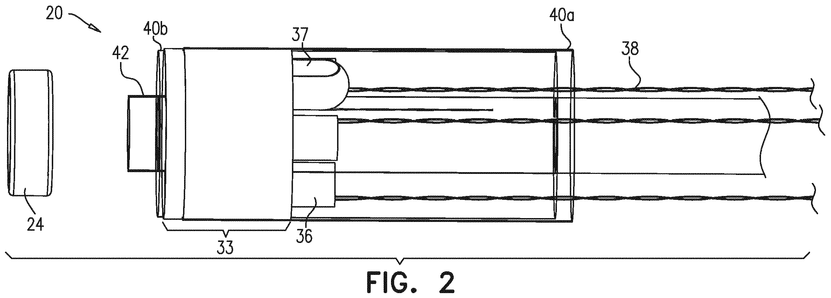

Fig. 1 depicts a physician 34 using catheter 22 to ablate cardiac tissue of a subject 26, by passing ablating currents, which are generated by a signal generator (SIG GEN) 28, into the tissue, while the catheter is inside the body of the subject. To perform this procedure, physician 34 first navigates the catheter to the heart 25 of subject 26. Subsequently, the physician passes the ablating signals, from an ablation electrode 21 that is coupled distally to a distal portion 29 of the catheter, into the tissue of heart 25. During the procedure, irrigating fluid, supplied by a pump 31, may be delivered via the catheter to electrode 21 (as further described below with reference to Figs. 2-3), and passed through apertures in the electrode.

Apparatus 20 comprises a conducting element 24 that is held by distal portion 29 of the catheter. For example, conducting element 24 may be held within distal portion 29, and/or affixed to the distal end of distal portion 29. The conducting element may comprise a plate, a tube, or any other suitably-shaped electrically-conductive piece of material.

Typically, distal portion 29 of the catheter is of enhanced flexibility, relative to the more proximal portions of the catheter. (In this context, "flexibility" includes both transverse flexibility and axial flexibility, e.g., compressibility.) For example, distal portion 29 may be shaped to define at least one groove, such as a helical groove 27, that provides enhanced flexibility. Alternatively or additionally, distal portion 29 may be made of a more flexible material than the more proximal portions of the catheter. The enhanced flexibility of portion 29 increases the response of portion 29 to any mechanical forces applied thereto, such that the position and/or orientation of conducting element 24 experience relatively large changes as mechanical forces are applied to the catheter. Typically, a protective flexible tube (not shown) is placed over distal portion 29.

In some embodiments, distal portion 29 comprises a flexible distal portion of the main tube 23 of catheter 22, which is of enhanced flexibility relative to the more proximal portion of tube 23. For example, distal portion 29 may comprise a distal, grooved portion of tube 23. In other embodiments, as shown in Fig. 1, distal portion 29 comprises a cylindrical element 29a that extends distally from tube 23, e.g., for a distance L of between 0.5 and 2 mm. (For example, during the manufacturing process, cylindrical element 29a may be inserted into tube 23, such that the proximal end of the cylindrical element is held by tube 23.) Cylindrical element 29a is of enhanced flexibility relative to tube 23, e.g., by virtue of being grooved, and/or by virtue of being made of a more flexible material than tube 23. For example, cylindrical element 29a may comprise a grooved stainless steel tube, which may also be referred to as a "spring."

It is noted that apparatus 20 may comprise any other suitable components, such as one or more sensing electrodes, alternatively or additionally to ablation electrode 21. Alternatively to using catheter 22 for an ablation procedure, the physician may use the catheter for any other suitable procedure within heart 25 (such as an electroanatomical mapping), or within any other portion of the body of subject 26.

Typically, the proximal end of catheter 22 is connected, via an electronic interface 46, to a console 48, which comprises, in addition to signal generator 28 and pump 31, a processor (PROC) 30. Electronic interface 46 may include any suitable circuitry, such as analog-to-digital (A/D) and digital-to-analog (D/A) converters. During the procedure, processor 30 generates digital signals that are converted, by interface 46, to analog signals. These signals are applied across a transmitting coil (Figs. 2-3) located near conducting element 24, causing the transmitting coil to generate an alternating magnetic field. This magnetic field induces eddy currents in the conducting element, which are detected by receiving coils (Figs. 2-3) near the conducting element. The receiving coils generate analog signals, which are converted to digital signals by interface 46. Processor 30 receives the digital signals and, by analyzing these signals, ascertains the magnitude and direction of the mechanical forces acting on the catheter, as further described below with reference to Figs. 2-3.

In general, processor 30 may be embodied as a single processor, or as a cooperatively networked or clustered set of processors. Processor 30 is typically a programmed digital computing device comprising a central processing unit (CPU), random access memory (RAM), non-volatile secondary storage, such as a hard drive or CD ROM drive, network interfaces, and/or peripheral devices. Program code, including software programs, and/or data are loaded into the RAM for execution and processing by the CPU and results are generated for display, output, transmittal, or storage, as is known in the art. The program code and/or data may be downloaded to the computer in electronic form, over a network, for example, or it may, alternatively or additionally, be provided and/or stored on non-transitory tangible media, such as magnetic, optical, or electronic memory. Such program code and/or data, when provided to the processor, produce a machine or special-purpose computer, configured to perform the tasks described herein.

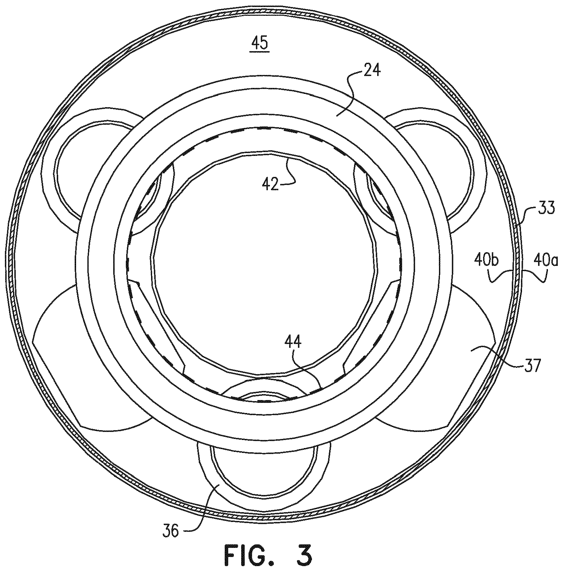

Reference is now made to Figs. 2-3, which show various components of apparatus 20 contained within catheter 22, in accordance with some embodiments of the present invention. Fig. 2 shows a side view of these components, while Fig. 3 shows a head-on view of these components. For clarity, in Figs. 2-3, tube 23, distal portion 29, and ablation electrode 21 are hidden from view.

As shown in Figs. 2-3, apparatus 20 comprises a transmitting coil 33, which is disposed, within catheter 22, proximally to conducting element 24, and typically also proximally to distal portion 29, e.g., at a distance of between 0.5 and 2 mm from the conducting element. Typically, a first electrically-insulating sheath 40a is disposed between the transmitting coil and tube 23.

Apparatus 20 further comprises a plurality of receiving coils 36, such as three or more receiving coils 36. Each of the receiving coils may be oriented axially, laterally, or in any other suitable orientation with respect to the longitudinal axis of catheter 22. In some embodiments, apparatus 20 further comprises one or more external-magnetic-field-sensing coils 37, which generate signals indicative of the position and orientation of the catheter in response to an external magnetic field. (It is noted that although Figs. 2-3 depict the various coils as solid structures, each of the coils actually comprises a tightly-wound wire.)

In some embodiments, as shown, transmitting coil 33 is ring shaped, in that the transmitting coil is shaped to define a central aperture 45. In such embodiments, receiving coils 36 may be disposed at least partly within aperture 45, i.e., within the transmitting coil. For example, as described above in the Overview, the transmitting coil may be wrapped around the receiving coils. Typically, in such embodiments, a second electrically-insulating sheath 40b is disposed between the receiving coils and the transmitting coil.

As described above with reference to Fig. 1, while catheter 22 is inside the body of the subject (e.g., while the ablating currents are passed into the tissue of the subject), an alternating voltage is applied across the transmitting coil. In some embodiments, this voltage has an amplitude of between 10 and 300 mV, and/or a frequency of between 3 and 20 kHz. The alternating voltage causes transmitting coil 33 to generate an alternating magnetic field, which induces eddy currents in conducting element 24. The eddy currents generate a secondary magnetic field that is superposed onto the magnetic field generated by the transmitting coil, thus producing a composite magnetic field. (In particular, the secondary magnetic field opposes the magnetic field generated by the transmitting coil, such that, for example, a higher-magnitude secondary magnetic field implies a lower-lower magnitude composite magnetic field.) The composite magnetic field induces a voltage in each of the receiving coils, such that each of the receiving coils outputs a respective current or voltage signal responsively to the composite magnetic field.

As further described above with reference to Fig. 1, the flexible distal portion flexes in response to the mechanical forces that are applied to the flexible distal portion of the catheter. By virtue of being held by the flexible distal portion, the conducting element moves as the flexible distal portion flexes, such that a force applied to the flexible distal portion of the catheter may cause the position and/or orientation of the conducting element to change. This change, in turn, causes a change in the induced eddy currents, and hence, in the voltages induced in the receiving coils.

Since the receiving coils are at different respective positions, and/or are oriented differently from each other, the position and orientation of the conducting element is a deterministic function of the induced voltages. Hence, processor 30 may ascertain the position and orientation of the conducting element from the signals that are output by the receiving coils. Given the position and orientation of the conducting element, the processor may further ascertain the magnitude and direction of the mechanical forces acting on the catheter. For example, the processor may use a function, or a lookup table, that maps the position and orientation of the conducting element to the magnitude and direction of the mechanical forces. Such a function or lookup table may be learned during a calibration procedure, in which the position and orientation of the conducting element is recorded while the catheter is subjected to controlled forces of various magnitudes and directions.

For example, as a mechanical force compresses the distal portion of the catheter, causing the conducting element to move closer to transmitting coil 33, higher eddy currents are induced in the conducting element, such that a higher secondary magnetic field opposes the field generated by the transmitting coil. Consequently, a lower voltage is induced in each of the receiving coils. In response to this lower voltage, processor 30 may ascertain the new position of the conducting element, and hence, the magnitude of the compressive force.

Typically, wires 38 run through the catheter, between the proximal end of the catheter and the coils. Wires 38 deliver electrical signals from interface 46 to transmitting coil 33, such that the transmitting coil may generate an alternating magnetic field while catheter 22 is within the body of the subject. Wires 38 further deliver output signals from receiving coils 36 to interface 46.

The ablation procedure described above with reference to Fig. 1 requires that the ablating currents be carried from signal generator 28 to ablation electrode 21 (Fig. 1). Moreover, some applications may require that a fluid-delivery tube 42 deliver irrigating fluid from pump 31 to the ablation electrode. Alternatively or additionally, one or more physiological sensors coupled to the catheter distally to the flexible distal portion of the catheter and conducting element 24, such as a temperature sensor (e.g., a thermocouple) or sensing electrode, may output signals, indicating physiological parameters of the subject, that must be carried to the proximal end of the catheter.

Hence, in some embodiments, conducting element 24 is shaped to define a central aperture (demarcated in the figure by a broken line 44) that allows passage therethrough of tubes, wires, and/or other elements. For example, conducting element 24 may be ringshaped or tube-shaped, with a diameter of, for example, 1.5-2.5 mm, and/or a thickness or length of 1-2 mm. Thus, for example, fluid-delivery tube 42 may pass through the central aperture of the conducting element, such that the fluid-delivery tube may deliver fluid, through the central aperture, to the ablation electrode. Alternatively or additionally, wires connected to the aforementioned physiological sensors, such as thermocouple wires, and/or wires that carry the ablating currents, may pass through the conducting element.

In other embodiments, conducting element 24 is closed, such that no tubes, wires, or other elements pass through the conducting element. In such embodiments, any required tubes, wires, or other elements may run alongside the conducting element.

In some embodiments, apparatus 20 comprises exactly one receiving coil. In such embodiments, apparatus 20 may comprise three or more transmitting coils that transmit at different respective frequencies, such that the magnitude and direction of any mechanical forces may be ascertained by the processor as described above. Alternatively, even if apparatus 20 comprises only a single receiving coil, apparatus 20 may comprise exactly one transmitting coil, and the processor may ascertain the magnitude of any mechanical forces in only a single direction, such as the axial direction.

## Claims

1. Apparatus, comprising:
a catheter (22) configured for insertion into a body of a subject, the catheter comprising a flexible distal portion (29) configured to flex in response to a mechanical force applied to the catheter, **characterised in that** it further comprises a conducting element (24), held by the flexible distal portion of the catheter such that a position of the conducting element changes as the flexible distal portion flexes;
at least one transmitting coil (33), disposed within the catheter proximally to the conducting element, configured to generate an alternating magnetic field that induces, in the conducting element, eddy currents that vary with the position of the conducting element; and
one or more receiving coils (36), disposed within the catheter proximally to the conducting element, configured to output respective signals responsively to a superposition of (i) the magnetic field generated by the transmitting coil, and (ii) a secondary magnetic field generated by the eddy currents.

2. The apparatus according to claim 1, wherein the conducting element is held within the flexible distal portion of the catheter, or is affixed to a distal end of the flexible distal portion of the catheter.

3. The apparatus according to claim 1, wherein the catheter further comprises a tube, and wherein the flexible distal portion of the catheter comprises a flexible distal portion of the tube that is of enhanced flexibility relative to a more proximal portion of the tube.

4. The apparatus according to claim 1, wherein the catheter further comprises a tube, and wherein the flexible distal portion of the catheter comprises a cylindrical element that extends distally from the tube and is of enhanced flexibility relative to the tube.

5. The apparatus according to claim 4, wherein the cylindrical element extends distally from the tube for a distance of between 0.5 and 2 mm.

6. The apparatus according to claim 1, wherein the flexible distal portion of the catheter is of enhanced flexibility by virtue of being shaped to define at least one groove.

7. The apparatus according to claim 6, wherein the at least one groove includes a helical groove.

8. The apparatus according to claim 1, further comprising a processor, configured to ascertain a magnitude and a direction of the mechanical force in response to the respective signals output by the receiving coils.

9. The apparatus according to claim 8, further comprising an electronic interface, wherein the processor is further configured to generate a digital signal, and wherein the electronic interface is configured to convert the digital signal to an analog signal which, when applied across the transmitting coil, causes the transmitting coil to generate the alternating magnetic field.

10. The apparatus according to claim 1, wherein the receiving coils are disposed at least partly within the transmitting coil.

11. The apparatus according to claim 10, wherein the transmitting coil is wrapped around the receiving coils.

12. The apparatus according to claim 1, wherein the conducting element comprises a plate or a tube.

13. The apparatus according to claim 1, wherein the conducting element is shaped to define a central aperture.

14. The apparatus according to claim 13, further comprising:
an ablation electrode, coupled distally to the flexible distal portion of the catheter, configured to pass ablating currents into tissue of the subject while the catheter is inside the body of the subject; and
a fluid-delivery tube that passes through the central aperture and is configured to deliver fluid to the ablation electrode.

15. The apparatus according to claim 13, further comprising:
at least one physiological sensor, coupled to the catheter distally to the flexible distal portion of the catheter; and
at least one wire that passes through the central aperture and is connected to the physiological sensor.

## Patentansprüche

1. Vorrichtung, umfassend:
einen Katheter (22), der zum Einführen in einen Körper eines Subjekts ausgestaltet ist, wobei der Katheter einen flexiblen distalen Abschnitt (29) umfasst, der ausgestaltet ist, um sich in Reaktion auf eine mechanische Kraft zu biegen, die auf den Katheter ausgeübt wird,
**dadurch gekennzeichnet, dass** er des Weiteren umfasst:
ein leitfähiges Element (24), das durch den flexiblen distalen Abschnitt des Katheters gehalten wird, so dass eine Position des leitfähigen Elements sich ändert, wenn der flexible distale Abschnitt gebogen wird;
mindestens eine Sendespule (33), die innerhalb des Katheters proximal des leitfähigen Elements angeordnet ist und ausgestaltet ist, um ein magnetisches Wechselfeld zu generieren, das in dem leitfähigen Element Wirbelströme induziert, die mit der Position des leitfähigen Elements varieren;
und
eine oder mehrere Empfangsspulen (36), die innerhalb des Katheters proximal des leitfähigen Elements angeordnet sind und ausgestaltet sind, um jeweilige Signale in Reaktion auf eine Überlagerung von (i) dem Magnetfeld, das durch die Sendespule generiert worden ist, und (ii) einem Sekundärmagnetfeld auszugeben, welches durch die Wirbelströme generiert worden ist.

2. Vorrichtung nach Anspruch 1, wobei das leitfähige Element innerhalb des flexiblen distalen Abschnitts des Katheters gehalten wird oder an einem distalen Ende des flexiblen distalen Abschnitts des Katheters angebracht ist.

3. Vorrichtung nach Anspruch 1, wobei der Katheter des Weiteren ein Rohr umfasst, und wobei der flexible distale Abschnitt des Katheters einen flexiblen distalen Abschnitt des Rohrs umfasst, der relativ zu einem weiter proximal liegenden Abschnitt des Rohrs eine erhöhte Flexibilität aufweist.

4. Vorrichtung nach Anspruch 1, wobei der Katheter des Weiteren ein Rohr umfasst, und wobei der flexible distale Abschnitt des Katheters ein zylindrisches Element umfasst, das sich distal des Rohrs erstreckt und relativ zu dem Rohr eine erhöhte Flexibilität aufweist.

5. Vorrichtung nach Anspruch 4, wobei das zylindrische Element sich über eine Distanz zwischen 0,5 und 2 mm distal des Schlauchs erstreckt.

6. Vorrichtung nach Anspruch 1, wobei der flexible distale Abschnitt des Katheters eine erhöhte Flexibilität aufweist, da er so geformt ist, dass er mindestens eine Nut definiert.

7. Vorrichtung nach Anspruch 6, wobei die mindestens eine Nut eine Spiralnut einschließt.

8. Vorrichtung nach Anspruch 1, des Weiteren umfassend einen Prozessor, der ausgestaltet ist, um einen Betrag und eine Richtung der mechanischen Kraft in Reaktion auf die jeweiligen Signale festzustellen, die von den Empfangsspulen ausgegeben werden.

9. Vorrichtung nach Anspruch 8, des Weiteren umfassend eine elektronische Schnittstelle, wobei der Prozessor des Weiteren ausgestaltet ist, um ein digitales Signal zu generieren, und wobei die elektronische Schnittstelle ausgestaltet ist, um das digitale Signal in ein analoges Signal zu konvertieren, das, wenn es über der Sendespule angelegt wird, bewirkt, dass die Sendespule das magnetische Wechselfeld generiert.

10. Vorrichtung nach Anspruch 1, wobei die Empfangsspulen mindestens teilweise innerhalb der Sendespule angeordnet sind.

11. Vorrichtung nach Anspruch 10, wobei die Sendespule um die Empfangsspulen herum gewickelt ist.

12. Vorrichtung nach Anspruch 1, wobei das leitfähige Element eine Platte oder ein Rohr umfasst.

13. Vorrichtung nach Anspruch 1, wobei das leitfähige Element so geformt ist, dass es einen zentralen Durchlass definiert.

14. Vorrichtung nach Anspruch 13, des Weiteren umfassend:
eine Ablationselektrode, die distal an den flexiblen distalen Abschnitt des Katheters gekoppelt ist und ausgestaltet ist, um Ablationsströme in Gewebe des Subjekts zu führen, während sich der Katheter innerhalb des Körpers des Subjekts befindet; und
ein Fluidabgaberohr, welches durch den zentralen Durchlass geführt wird und ausgestaltet ist, um Fluid an die Ablationselektrode abzugeben.

15. Vorrichtung nach Anspruch 13, des Weiteren umfassend:
mindestens einen physiologischen Sensor, der distal des flexiblen distalen Abschnitts des Katheters an den Katheter gekoppelt ist; und
mindestens einen Draht, der durch den zentralen Durchlass führt und mit dem physiologischen Sensor verbunden ist.

## Revendications

1. Appareil, comprenant :
un cathéter (22) configuré pour une insertion dans le corps d'un sujet, le cathéter comprenant une partie distale souple (29) configurée pour fléchir en réponse à une force mécanique appliquée au cathéter, **caractérisé en ce qu'**il comprend en outre un élément conducteur (24) maintenu par la partie distale souple du cathéter de telle sorte qu'une position de l'élément conducteur change lorsque la partie distale souple fléchit ;
au moins une bobine de transmission (33) disposée à l'intérieur du cathéter à proximité de l'élément conducteur, configurée pour générer un champ magnétique alternatif qui induit, dans l'élément conducteur, des courants de Foucault qui varient avec la position de l'élément conducteur ; et
une ou plusieurs bobines de réception (36), disposées à l'intérieur du cathéter à proximité de l'élément conducteur, configurées pour délivrer en sortie des signaux respectifs en réponse à une superposition (i) du champ magnétique généré par la bobine de transmission, et (ii) d'un champ magnétique secondaire généré par les courants de Foucault.

2. Appareil selon la revendication 1, l'élément conducteur étant maintenu dans la partie distale souple du cathéter, ou étant fixé à une extrémité distale de la partie distale souple du cathéter.

3. Appareil selon la revendication 1, le cathéter comprenant en outre un tube, et la partie distale souple du cathéter comprenant une partie distale souple du tube qui est d'une flexibilité accrue par rapport à une partie plus proximale du tube.

4. Appareil selon la revendication 1, le cathéter comprenant en outre un tube, et la partie distale souple du cathéter comprenant un élément cylindrique qui s'étend de manière distale à partir du tube et qui est d'une flexibilité accrue par rapport au tube.

5. Appareil selon la revendication 4, l'élément cylindrique s'étendant de manière distale par rapport au tube sur une distance comprise entre 0,5 et 2 mm.

6. Appareil selon la revendication 1, la partie distale souple du cathéter présentant une flexibilité accrue du fait qu'elle est formée de manière à définir au moins une rainure.

7. Appareil selon la revendication 6, l'au moins une rainure comprenant une rainure hélicoïdale.

8. Appareil selon la revendication 1, comprenant en outre un processeur, configuré pour déterminer une amplitude et une direction de la force mécanique en réponse aux signaux respectifs délivrés en sortie par les bobines de réception.

9. Appareil selon la revendication 8, comprenant en outre une interface électronique, le processeur étant en outre configuré pour générer un signal numérique, et l'interface électronique étant configurée pour convertir le signal numérique en un signal analogique qui, lorsqu'il est appliqué à travers la bobine de transmission amène la bobine d'émission à générer le champ magnétique alternatif.

10. Appareil selon la revendication 1, les bobines de réception étant disposées au moins en partie à l'intérieur de la bobine d'émission.

11. Appareil selon la revendication 10, la bobine d'émission étant enroulée autour des bobines de réception.

12. Appareil selon la revendication 1, l'élément conducteur comprenant une plaque ou un tube.

13. Appareil selon la revendication 1, l'élément conducteur étant formé de manière à définir une ouverture centrale.

14. Appareil selon la revendication 13, comprenant en outre :
une électrode d'ablation, couplée de manière distale à la partie distale souple du cathéter, configurée pour faire passer des courants d'ablation dans le tissu du sujet tandis que le cathéter est à l'intérieur du corps du sujet ; et
un tube de distribution de fluide qui passe à travers l'ouverture centrale et est configuré pour acheminer le fluide jusqu'à l'électrode d'ablation.

15. Appareil selon la revendication 13, comprenant en outre :
au moins un capteur physiologique, couplé au cathéter de manière distale à la partie distale souple du cathéter ; et
au moins un fil qui passe à travers l'ouverture centrale et qui est connecté au capteur physiologique.
